Europäisches Patentamt

European Patent Office

Office européen des brevets

(11) Publication number: **0 216 467**
**A2**

## (12) EUROPEAN PATENT APPLICATION

(21) Application number: 86305743.6

(22) Date of filing: 25.07.86

(51) Int. Cl.⁴: **C12M 1/40**

(30) Priority: 20.09.85 US 778061

(43) Date of publication of application:
01.04.87 Bulletin 87/14

(84) Designated Contracting States:
AT BE CH DE FR GB IT LI LU NL SE

(71) Applicant: THE REGENTS OF THE UNIVERSITY
OF CALIFORNIA
2199 Addison Street
Berkeley California 94720(US)

(72) Inventor: Gough, David A.
1435 Big Canyon Terrace
Cardiff by the Sea California 92007(US)

(74) Representative: Wilson, Nicholas Martin et al
WITHERS & ROGERS 4 Dyer's Buildings
Holborn
London EC1N 2JT(GB)

(54) Two-dimensional diffusion glucose substrate sensing electrode.

(57) Enzyme electrode assembly suitable for sensing physiologically important molecules such as glucose wherein the rate of accessibility of enzyme substrates to the enzyme is differentially regulated by suitably positioning material with selective permeability properties around the sensing region of the electrode assembly.

EP 0 216 467 A2

# TWO-DIMENSIONAL DIFFUSION GLUCOSE SUBSTRATE SENSING ELECTRODE

This invention relates to a two-dimensional diffusion glucose substrate sensing electrode.

## BACKGROUND OF THE INVENTION

It is standard practice to treat diabetes mellitus predominately with injections of insulin to counter the inability of the pancreas to manufacture and secrete insulin in response to elevated blood glucose levels. For optimal therapy, it is necessary to determine the glucose concentration in the body in order to specify the appropriate amount and time of the antidiabetes medication. This requires a glucose sensing device, which may be most effective if implanted in the body.

One approach to the development of an implantable sensor is the so-called enzyme electrode in which an immobilized enzyme catalyzes a chemical reaction between glucose and another molecule such as oxygen that can be detected by an electrode. The enzymatic reaction is one in which glucose is catalytically converted to gluconic acid with the simultaneous consumption of oxygen promoted by the enzyme glucose oxidase, with the resulting decrease in oxygen determined by an amperometric oxygen electrode and thereby related to glucose levels. A key problem in developing this sensor is that the glucose concentration in the body is normally higher than the oxygen concentration by a factor of 50 -1000 times. Thus, since the enzymatic reaction is limited by oxygen, the least abundant reactant, glucose measurements in the body are often inaccurate. Most previous investigators have not recognized or gone beyond this problem.

Hicks et al. in U.S. Patent No. 3,542,662 describe an electrolytic glucose sensor capable of assaying glucose in fluid removed from the body, but not usable for measuring glucose directly in vivo. Hicks et al. describe an enzyme-containing membrane disposed between a fluid being assayed and first oxygen sensor electrode and a similar membrane not containing enzymes disposed between a fluid and second reference oxygen electrode. Oxygen diffuses through the enzyme-containing membrane and is consumed in an equal molar reaction with glucose catalyzed by the enzyme glucose oxidase; consequently, oxygen is unavailable for detection by the oxygen sensor electrode. The second oxygen sensor electrode measures the concentration of oxygen existing absent any enzyme-catalyzed reaction. The difference in oxygen levels detected by the two electrodes is proportional to the glucose concentration within certain limits.

The glucose sensor described by Hicks et al. performs satisfactorily when assaying for glucose in vitro. However, when the sensor is used directly in the body, its performance is unreliable. In part, this appears to be due to the inability of the dual-sensor design to function adequately in low-oxygen environments.

Presently there does not exist an implantable sensor suitable for detecting glucose in regions of the body where oxygen concentrations are lower than glucose concentrations. Previously, however, Fisher and Abel in "A Membrane Combination for Implantable Glucose Sensors, Measurements in Undiluted Biological Fluids" (Trans. Am. Soc.Artif. Intern. Organs, Vol. XXVIII, 1982) have attempted to solve the problem by positioning sandwich membranes in association with an oxygen electrode sensor. The membranes consist of a hydrophobic layer over an enzyme layer, with the former having a minute hole aligned with the oxygen electrode sensor so as to allow predominately access of glucose from the fluid being assayed. The hydrophobic layer was selected to be permeable predominantly to oxygen; consequently, oxygen diffuses into the enzyme layer at all points across the surface of the hydrophobic layer whereas glucose diffuses in only at the region of the hole. In this manner, a stoichiometric excess of oxygen over glucose was provided to the enzyme layer. As a result of the requirement that glucose entry be restricted to a small opening in the hydrophobic membrane, this oxygen electrode sensor apparatus is limited as to the range of concentrations of glucose detectable. Also, because the enzyme situated near the opening in the hydrophobic membrane is constantly exposed to incoming glucose, it tends to become inactivated, which in turn requires that for the sensor to continue functioning that glucose diffuse further into the membrane, which additionally limits the use of the sensor in that it increases its response time.

## BRIEF SUMMARY OF THE INVENTION

An oxygen electrode sensor is described that can be implanted in the body, and which is suitable for detecting glucose in regions of the body where oxygen concentrations are stoichiometrically less than glucose concentrations. The sensor consists of a hydrophilic layer containing enzyme in communication with the sensor and a hydrophobic layer in communication with the hydrophilic layer. The hydrophilic and hydrophobic layers are positioned so that the enzyme substrates, glucose and oxygen diffuse predominantly in a two-directional manner such that the substrates enter the hydrophilic layer at right angles to each other. Oxygen enters the enzyme region by diffusion through the hydrophobic layer and, to a lesser extent, through the exposed surface of the hydrophilic layer; but glucose, being incapable of diffusion in the hydrophobic layer, enters only through the exposed surface of the hydrophilic layer. By establishing a two-directional diffusion gradient of substrates, it is possible to effectively increase the relative oxygen concentration entering the enzyme region. Additionally, a second oxygen sensor electrode capable of acting as a reference electrode to measure the level of ambient oxygen present in the absence of enzymatic catalysis of glucose is described.

## BRIEF DESCRIPTION OF THE DRAWINGS

Reference is made to the drawings illustrating various embodiments of the invention in which:

FIGURE 1 is a schematic diagram of one form of the present invention;

FIGURE 2 is a schematic presentation of a second form of the present invention;

FIGURE 3 is a still further form - schematically representing the present invention.

The invention will be described as applied to determining concentrations of glucose in bodily fluids containing a large stoichiometric excess of glucose over oxygen. It will be understood, however, by those skilled in the arts that the electrode assembly of the present invention is not limited to assay solely glucose, and that it may be used to assay other molecules such as amino acids, lactates, ammonia, or similar molecules commonly found in bodily fluids that are substrates for oxidase enzymes that require the presence of a gaseous species to undergo enzymatic conversion.

An electrode assembly suitable for detecting concentrations of glucose in bodily fluids where the electrode assembly detects a change in oxygen due to the conversion of glucose to gluconic acid is constructed of three parts an electrochemical oxygen sensor having the appropriate geometry, a gelatinous layer made of hydrophilic material, and a hydrophobic layer. The gelatinous hydrophilic material contracts the sensor part of the assembly. Contained within, or associated with, the gelatinous material is a enzyme, glucose oxidase, and optionally a second enzyme, catalase. The oxidase enzyme promotes the following reaction:

glucose + oxygen + water → gluconic acid + hydrogen peroxide

Hydrogen peroxide can be decomposed by incorporating catalase in the gelatinous material should it be desirable to do so. Catalase catalyzes the following reaction:

$$H_2O_2 \rightarrow \tfrac{1}{2}O_2 + H_2O$$

Materials useful for preparing the gelatinous layer include polyacrylamide gels, glutaraldehyde - cross-linked collagen or albumin, polyhdroxyethylmethacrylate, and its derivatives and other hydrophilic polymers and copolymers. The layer can similarly be constructed by cross-linking glucose oxidase or other enzymes with chemical cross-linking reagents.

In association with the gelatinous hydrophilic layer, is a second layer made of hydrophobic material. Materials suitable for constructing the second layer are polydimethylsiloxane, polymers of tetrafluoroethylene, or its fluorocarbon analogs alone or as copolymers with ethylene or propylene, polyethylene, polypropylene, cellulose acetate, and other oxygen-imbibing polymeric materials. Generally, these materials will account for about 2% - 40% of the combined weight of the hydrophilic and hydrophobic layers.

The hydrophilic gel layer containing the enzyme will most often be disposed between a thin layer of the hydrophobic material covering a portion or all of the face of the hydrophilic layer and the oxygen sensor but leaving the edge surfaces, due to the thickness of that layer, accessible to the solution to be analyzed. The hydrophilic gelatinous layer is permeable to both glucose and gaseous molecules, such as oxygen. Glucose oxidase impregnated in the hydrophilic layer acts on glucose and oxygen as they diffuse through it.

The hydrophobic material of the second layer is relatively impermeable to glucose but permeable to oxygen. Since the conversion of glucose to gluconic acid is limited stoichiometrically by whichever component is present at the oxygen-sensitive electrode in lowest concentration, in order to make the electrode assembly sensitive to glucose concentrations oxygen within the enzyme region must be at least stoichiometrically equal to glucose. This requirement is met by covering they hydrophilic gel layer with the hydrophobic layer, which hinders the rate of entry of glucose but provides access of oxygen to the gel layer. Thus, by disposing the hydrophobic layer in a body or bodies that limits the surface area of the hydrophilic gel layer exposed for accepting glucose from bodily fluids causes a reduced rate of entry of glucose into the hydrophilic gel layer, which decreases the concentration of glucose inside the hydrophilic gelatinous layer to a concentration that allows for stoichiometric reaction with oxygen.

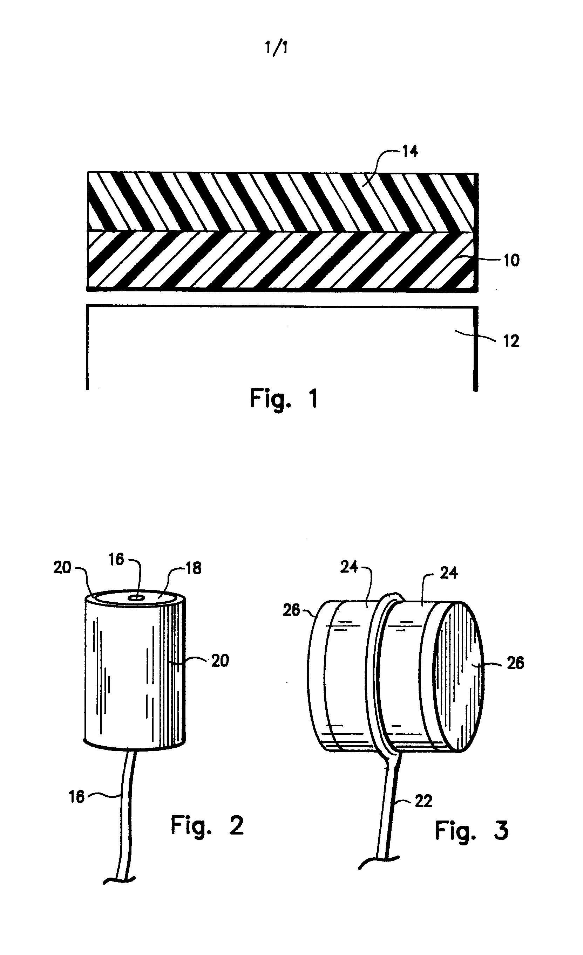

In order to efficaciously measure glucose in low oxygen concentrations in the body, the first and second layers of the electrode assembly, hydrophilic and hydrophobic layers, respectively, can be positioned relative to each other in several ways. In the electrode assembly shown in Figure 1, glucose enters the membrane only through the edge surfaces of the gelatinous hydrophilic layer 10 and diffuses toward the centre portion of the layer substantially parallel to the face of the surface of the layer and the surface of the planar sensor 12. The gaseous species, oxygen, enters the hydrophobic membrane 14 through the entire exposed surface of the hydrophobic layer and subsequently penetrates into the gelatinous hydrophilic layer to insure that there is an excess of oxygen over glucose.

An additional configuration of the hydrophilic and hydrophobic layer is shown in Figure 2. Here the oxygen sensor portion 16 of the assembly exhibits a cylindrical shape with its active surface being the curve of cylindrical face rather than the flat end. A concentric hydrophilic layer 18 contains the enzymes that contacts the oxygen sensor portion along the curved surface and a concentric hydrophobic layer 20 contacts the curved face but not the flat face of the hydrophilic layer. In this arrangement, the glucose enters only through the flat hydrophilic exposed edge and oxygen enters predominantly through the curved sides of the hydrophobic layer.

A third arrangement shown in Figure 3 is a combination of a circular-plate oxygen sensor 22 sensitive to oxygen on one or both of its flat surfaces but not at the curved edges with a hydrophilic enzyme-containing layer 24 contacting the oxygen sensor on each side and with a hydrophobic layer 26 contacting the outer surface of each hydrophilic layer but not the circular edge. Thus, oxygen predominantly enters from both sides through the two hydrophilic layers in a direction perpendicular to the plane while glucose enters only through the hydrophilic circumferential edge.

To insure operation of the sensor assembly to respond to glucose at low relative external concentrations of oxygen, the hydrophilic layer containing glucose oxidase is designed to so that oxygen passes readily into it through the exposed edge and through the contacting hydrophobic layer whereas glucose, diffuses into it only through the exposed edge and not through the hydrophobic layer. The ratio of oxygen to glucose is thus controlled by (1) the external concentrations, (2) the entry and/or transport properties of the combination of hydrophilic and hydrophobic layers, and (3) the aspect ratio, or area of the exposed hydrophilic surface available for glucose transport to the area of the hydrophobic surface available to only oxygen transport. The relative proportions of glucose and oxygen are thus changed from a concentration ratio in the body between approximately 50 -1000 parts of glucose to 1 part of oxygen to a new ratio in which a slight stoichiometric excess of oxygen exists in the hydrophilic layer. Consequently, the electrode assembly is not stoichiometrically limited by the concentration of oxygen in bodily fluids, and the system operates in a manner that is sensitive to the glucose concentration being measured.

The following example is given to aid in understanding the invention, but it is to be understood that the invention is not limited to the particular materials or procedures of the example.

EXAMPLE I

A membrane-covered cylindrical oxygen sensor was fashioned from two platinum wires and a silver wire cemented into a glass bead and coated with a hydrophobic polymer. The opposite wire ends protruding through the glass bead were connected to the electrochemical analysis instrumentation. The exposed flat end of the oxygen sensor was rendered electro-chemically inactive by the application of a thin impermeable coating. a segment of silicone rubber tubing, 0.07ins. I.D. x 0.11 ins. O.D., (Dow Corning Corporation) was fitted over the sensor and cemented to the glass bead at the closed end leaving a concentric cavity. The enzymes were immobilized in a gel formed in the cylindrical cavity between the sensor and the tube. The gel contained 20gm% denatured bovine achilles tendon collagen, 6gm% glucose oxidase from A. niger(Sigma Chemical Company, type VII), and catalase. These gel components were dissolved in 0.1M phosphate buffer, pH 7.3, and cross-linked with glutaraldehyde (25% solution).

The resulting glucose sensor was placed in a sealed, thermostated (37°C) vessel containing 0.1M phosphate buffer, pH 7.3, and equilibrated with the gas mixture containing 1.8% oxygen - (0.02mM).

An identical oxygen sensor without the enzyme was used to indicate the ambient oxygen concentration. Aliquots of concentrated glucose were added and the following results obtained.

## Table 1.  Sensor Response to Glucose

| Glucose Concentration (mM) | 0.0 | 0.25 | 0.50 | 1.00 | 4.00 | 8.50 |
|---|---|---|---|---|---|---|
| Sensor Signal (%) (Glucose electrode signal normalized by oxygen reference electrode signal) | 0 | 48 | 54 | 62 | 75 | 95 |

## Claims

1. A sensor assembly suitable for detecting physiologically important large molecules in the body comprising a base sensor for detecting small molecules dissolved in bodily fluids,

a permeable first layer accessible to said bodily fluids and in communication with said base sensor and containing at least one catalyst or enzyme therein,

a permeable second layer accessible to said bodily fluids and contacting said permeable first layer to provide differential diffusion of said physiologically important large molecules and said small molecules into said permeable first layer and thereby allowing for stoichiometric amounts of said physiologically important large molecules and said small molecules to react with said catalyst or enzyme in said permeable first layer, thereby altering the concentration of said small molecule detected by said base sensor,

a second base sensor not containing catalyst or enzyme for determining the background concentration of said small molecule thereby providing a means of determining the concentration of any unreacted small molecules or reaction products of said small molecules and/or said physiologically important large molecules.

2. A sensor assembly as described in Claim 1 wherein said permeable first layer is permeable to glucose and oxygen.

3. A sensor assembly as described in Claim 1 wherein said second layer is permeable to oxygen and relatively impermeable to glucose.

4. A sensor assembly as described in Claim 1 wherein said base sensor is an oxygen or hydrogen peroxide detector.

5. A sensor assembly as described in Claim 1 wherein said physiologically important large molecules are drawn from the group consisting of glucose, lactates, amino acids, and alcohol.

6. A sensor assembly as described in Claim 1 wherein said small molecules are oxygen or hydrogen peroxide.

7. A sensor assembly as described in Claim 1 wherein said enzyme is glucose oxidase and/or catalase.

8. An electrode assembly as described in Claim 1 wherein said first layer is fabricated from materials drawn from the group consisting of polyacrylamide, glutaraldehyde -cross-linked collagen or albumen, polyhydroxyethylmethacrylate and its derivatives, and other hydrophilic proteins, polymers, and copolymers.

9. A sensor assembly as described in Claim 1 wherein said second layer is made of material drawn from the group consisting of polydimethylsiloxynane, polymers of tetrafluroethylene, or its fluoro-chloro analogs alone or as copolymers with ethylene or propylene, polyethylene, polypropylene, cellulose acetate, and other oxygen-imbibing polymeric materials.

10. A sensor assembly as described in Claim 1 wherein said second layer contacts said first layer so that the surface area of said second layer is exposed to said bodily fluids in an amount greater than the surface area of said first layer exposed to said bodily fluids to provide at least a stoichiometric equivalent of said physiologically important large molecules to said small molecule in said first layer.

11. A method for measuring physiologically important large molecules in the body comprising:

a sensor assembly suitable for measuring physiologically important large molecule in the body comprising a base sensor for detecting small molecules dissolved in bodily fluids,

a permeable first layer accessible to said bodily fluids and in communication with said base sensor and containing at least one catalyst or enzyme therein,

a permeable second layer accessible to said bodily fluids and contacting said permeable first layer to provide differential diffusion of said physiologically important large molecules and said small molecules into said permeable first layer and thereby allowing for stoichiometric amounts of said physiologically important large molecules and said small molecules to react with said catalyst or enzyme in said permeable first layer thereby altering the concentration of said small molecule detected by said base sensor,

a second base sensor not containing catalyst or enzyme for determining the background concentration of said small molecule thereby providing a means of determining the concentration of any unreacted small molecules or reaction products of said small molecules and/or said physiologically important large molecules.

12. A method as defined in Claim 11 wherein said permeable first layer is permeable to glucose and oxygen.

13. A method as defined in Claim 11 wherein said second layer is permeable to oxygen and relatively impermeable to glucose.

14. A method as defined in Claim 11 wherein said base sensor is an oxygen or hydrogen peroxide detector.

15. A method as defined in Claim 11 wherein said physiologically important large molecules are drawn from the group consisting of glucose, lactates, amino acids, and alcohol.

16. A method as defined in Claim 11 wherein said small molecules are oxygen or hydrogen peroxide.

17. A method as defined in Claim 11 wherein said enzyme is glucose oxidase, lactate oxidase, and/or catalase.

18. A method as defined in Claim 11 wherein said first layer is fabricated from materials drawn from the group consisting of polyacrylamide, glutaraldehyde -cross-linked collagen or albumin, polyhydroxyethylmethacrylate and its derivatives, and other hydrophilic proteins, polymers, and copolymers.

19. A method as defined in Claim 11 wherein said second layer is made of material drawn from the group consisting of polydimethylsioxynane, polymers of tetrafluroethylene, or its fluoro-chloro analogs alone or as copolymers with ethylene or propylene, polyethylene, polypropylene, cellulose acetate, and other oxygen-imbibing polymeric materials.

20. A method as defined in Claim 11 wherein said second layer covers said first layer such that the surface area of said second layer is exposed to bodily fluids in an amount greater than the surface area of the first layer exposed to bodily fluids to provide at least a stoichiometric equivalent of said physiologically important large molecules to said small molecules in said first layer.

1/1

Fig. 1

Fig. 2

Fig. 3